# EUROPÄISCHE PATENTSCHRIFT

(11) **EP 2 785 418 B1**
(45) Veröffentlichungstag und Bekanntmachung des Hinweises auf die Patenterteilung: **09.01.2019**
(21) Anmeldenummer: 12775031.3
(22) Anmeldetag: 23.10.2012
(51) Int. Cl.: A61Q 5/06, A61K 8/891, A61K 8/04, A61Q 5/12, A61K 8/37, A61K 8/92, A61K 8/31, A61Q 5/00

(54) **GLANZBRINGENDES HAARBEHANDLUNGSMITTEL**
SHINE-PRODUCING HAIR TREATMENT AGENT
PRODUITS DE TRAITEMENT POUR DONNER DU BRILLANT AUX CHEVEUX

(30) Priorität: 28.11.2011 DE 102011087233
(43) Veröffentlichungstag der Anmeldung: 08.10.2014
(73) Patentinhaber: Henkel AG & Co. KGaA, 40589 Düsseldorf (DE)
(72) Erfinder: BERGEMANN, Uwe, 22459 Hamburg (DE); MÜLLER, Burkhard, 40221 Düsseldorf (DE); KAFTAN, Pamela, 22763 Hamburg (DE)
(86) Internationale Anmeldenummer: PCT/EP2012/070921
(87) Internationale Veröffentlichungsnummer: WO 2013/079259

(56) Entgegenhaltungen:
- EP-A1- 1 216 684
- EP-A2- 2 105 128
- WO-A2-2007/099434
- DE-A1-102007 063 352
- DE-A1-102008 033 106
- US-A1- 2007 166 254
- US-A1- 2010 215 785
- "Haskell Cosmética Natural", , 1. November 2007 (2007-11-01), Seite 1, XP055070263, Gefunden im Internet: URL:http://www.gnpd.com/sinatra/gnpd/searc h_results&search_id=FXxc4AJfJy/&item_id=81 5251 [gefunden am 2013-07-09]

## Beschreibung

Die vorliegende Erfindung betrifft Mittel zur kosmetischen Behandlung keratinhaltiger Fasern, insbesondere zur kosmetischen Haarbehandlung.

Unter keratinhaltigen Fasern werden im Sinne der vorliegenden Anmeldung alle tierischen Haare, z.B. Wolle, Rosshaar, Angorahaar, Pelze, Humanhaar, Federn und daraus gefertigte Produkte oder Textilien verstanden. Vorzugsweise handelt es sich bei den keratinischen Fasern jedoch um menschliche Haare.

Das menschliche Haar ist stets Umwelteinflüssen, wie UV-Strahlung, Wärme, Wasser und mechanischen Reizen, ausgesetzt. Darüberhinaus erfährt das Haar im Rahmen einer kosmetischen Haarbehandlung oftmals eine Modifikation an der Haaroberfläche (zum Beispiel durch Abscheidung von Pflegestoffen) oder der Haarstruktur (z.B. durch Pflegestoffe oder chemische Modifikation im Rahmen einer reduktiven oder oxidativen Haarbehandlung). Die Anwendung eines Trockenshampoos bewirkt beispielsweise neben einer Entfernung von Schmutz ebenso die Reduktion von Sebum auf der Haaroberfläche, was dem Haar zusammen mit der Ablagerung von Fettstoffen auf dem Haar ein leicht stumpfes Aussehen verleiht. Ein weiteres Beispiel bietet eine kosmetische leave-on Haarbehandlung, bei der das kosmetische Mittel nach der Applikation nicht vom Haar gespült wird und auf der Haarfaser verbleibt. Durch seine Präsenz auf der Haaroberfläche nimmt das leave-on Kosmetikum einen Einfluss auf den visuellen Eindruck des Haars. Um gegebenenfalls unerwünschten Effekten, wie z.B. Einbußen des natürlichen Haarglanzes, zu kompensieren, ist es ratsam, ein Kosmetikum mit z.B. glanzgebenden Wirkstoffen einzusetzen.

Die glanzgebenden Wirkstoffe scheiden sich im Rahmen einer leave-on Anwendung meist auf der Oberfläche des Haars ab, was einerseits zum erwünschten Glanz führt. Andererseits kann diese leave-on Glanzanwendung der Wirkung des zuvor oder gleichzeitig applizierten kosmetischen Mittels zuwiderlaufen. Werden beispielsweise Öle als Glanzwirkstoffe eingesetzt könne diese Öle das Haar beschweren. Eine temporäre Haarfestigung mit fixierenden Polymeren kann beispielsweise durch die Gegenwart von das Haar beschwerenden Glanzwirkstoffen weniger effektiv sein, da das beschwerte Haar durch sein Eigengewicht schneller aushängt und die aufgeprägte Frisur rasch verloren geht. Dies kann zusätzlich durch eine den fixierenden Polymerfilm erweichende Eigenschaft des Glanzwirkstoffs begünstigt werden.

Ausserdem kann die unmittelbare Kombination von Glanzwirkstoff und Trockenshampoo die Effektivität der Waschwirkung des Trockenshampoos erniedrigen.
Aufgabe der vorliegenden Erfindung war es daher, ein kosmetisches Mittel zur Pflege keratinischer Fasern zur Verfügung zu stellen, das einen hervorragenden Glanz auf den keratinhaltigen Fasern bewirkt ohne die Fasern zu beschweren.

Es wurde nunmehr gefunden, dass dies durch eine Kombination spezieller Öle mit speziellen Estern erreicht wird, insbesondere wenn sie als feiner Sprühnebel auf die Faser appliziert wird. Zudem wurde erreicht, dass sich die behandelten Fasern besser entwirren lassen, was sich in einer verbesserten Kämmbarkeit der keratinischen Fasern niederschlägt.

Ein erster Gegenstand der vorliegenden Erfindung ist daher die
Verwendung eines Mittels, enthaltend bezogen auf das Gesamtgewicht des Mittels
(i) 1,0 bis 10,0 Gew.-% mindestens eines Esters Benzoesäure mit einem C12 bis C15-Alkanol, und
(ii) 1,0 bis 15,0 Gew.-% mindestens eines Silikonöls der Formel (Si-1) (CH₃)₃Si-[O-Si(CH₃)₂]ₓ-O-Si(CH₃)₃ (Si-1), enthalten, in der x für eine Zahl von 0 bis 20 steht und
(iii) 30,0 bis 70,0 Gew.-% Ethanol, als Bestandteil des kosmetisch akzeptablen Trägers. zur Erzeugung von Glanz auf menschlichen Haaren.

Die Arylstruktureinheiten der Arylgruppe, Aryl(C₁ bis C₄)-alkylgruppe und Aryl-(C₂ bis C₄)-alkenylgruppe können Substituenten tragen. Bevorzugte Substituenten sind Hydroxy, Amino, (C₁ bis C₄)-alkylamino, Di(C₁ bis C₄)-alkylamino, (C₁ bis C₄)-Alkyl, (C₁ bis C₄)-Alkoxy, (C₁ bis C₄)-Alkoxycarbonyl. Beispiele für erfindungsgemäße gegebenenfalls substituierte Arylgruppen sind Phenyl, 2-Hydroxyphenyl, 2-Methoxyphenyl, Naphthyl, 7-Hydroxynaphthyl, 5-Hydroxynaphthyl. Beispiele für erfindungsgemäße Aryl-(C₁ bis C₄)-alkylgruppen sind Benzyl, 2-Phenylethyl. Beispiele für erfindungsgemäß Aryl-(C₂ bis C₄)-alkenylgruppen sind Styryl.
Beispiele für erfindungsgemäße (C₂ bis C₆)-Hydroxyalkylgruppen sind 1-Hydroxyethyl, 2-Hydroxyethyl, 2-Hydroxypropyl, 2-Hydroxybutyl, 3-Hydroxybutyl, 3-Hydroxy-2-methylpropyl.
Beispiele für eine bevorzugte eine (C₂ bis C₃₀)-Alkylpoly(oxyethylen)gruppe

Er ist erfindungsgemäß bevorzugt, wenn die erfindungsgemäßen Mittel als Sprühnebel, insbesondere als mit Hilfe eines Treibmittels erzeugte Sprühnebel, vorliegen.

Es ist erfindungsgemäß bevorzugt, wenn gemäß Formel (I) mindestens eine der Gruppen R¹ und R² eine gegebenenfalls substituierte Arylgruppe ist. Dabei ist es wiederum bevorzugt, wenn R² für eine gegebenenfalls substituierte Arylgruppe, insbesondere für gegebenenfalls substituiertes Phenyl, steht. Bevorzugte Substituenten der Aryl bzw. Phenylgruppe sind Hydroxy, Amino, (C₁ bis C₄)-alkylamino, Di(C₁ bis C₄)-alkylamino, (C₁ bis C₄)-Alkyl, (C₁ bis C₄)-Alkoxy, (C₁ bis C₄)-Alkoxycarbonyl.

Ganz besonders bevorzugt enthält das erfindungsgemäße Mittel als Ester der obigen Formel (I) mindestens einen Ester der Formel (I-a) 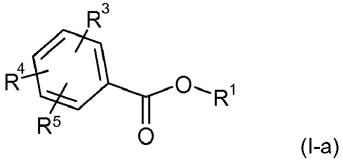 worin
- R¹: steht für eine lineare oder verzweigte (C₃ bis C₂₄)-Alkylgruppe, eine lineare oder verzweigte (C₆ bis C₂₄)-Alkenylgruppe, eine gegebenenfalls substituierte Arylgruppe, eine gegebenenfalls substituierte Aryl-(C₁ bis C₄)-alkylgruppe oder eine (C₂ bis C₆)-Hydroxyalkylgruppe, und
- R³, R⁴ und R⁵: stehen unabhängig voneinander für ein Wasserstoffatom, Hydroxy, Amino, (C₁ bis C₄)-alkylamino, Di(C₁ bis C₄)-alkylamino, (C₁ bis C₄)-Alkyl, (C₁ bis C₄)-Alkoxy, (C₁ bis C₄)-Alkoxycarbonyl, oder zwei der Reste bilden gemeinsam mit dem Restmolekül einen fünf- oder sechsgliedrigen Ring.

Ester der Benzoesäure mit einem (C₁₂ bis C₁₅)-Alkanol können beispielsweise unter dem Handelsnamen Finsolv® TN (INCI-Bezeichnung: C12-15 Alkyl Benzoate) von der Firma Finetex bezogen werden.

In dem Mittel sind die besagten Ester der Komponente (i), insbesondere die bevorzugt genannten besagten Ester der Komponente (i), in Bezug auf das Gesamtgewicht des erfindungsgemäßen Mittels in einer Menge von 0,5 bis 15,0 Gew.-%, insbesondere von 1,0 bis 10,0 Gew.-%, enthalten.

Als weitere Komponente (ii) enthält das Mittel zwingend ein Silikonöl der Formel (Si-1)

(CH₃)₃Si-[O-Si(CH₃)₂]ₓ-O-Si(CH₃)₃ (Si-1),

enthalten, in der x für eine Zahl von 0 bis 20 und insbesondere 0 bis 10 steht.

Diese Silikone werden nach der INCI-Nomenklatur als Hexamethyldisiloxane (Formel (Si-1) mit x=0), Trisiloxane (Formel (Si-1) mit x = 1) und Dimethicone (Formel (Si-1) mit x = 2-100) bezeichnet. Es werden im Rahmen der vorliegenden Erfindung als Silikon der Formel (Si-1) mindestens eine Verbindung aus (CH₃)₃Si-O-Si(CH₃)₃

| | |
|---|---|
| (CH₃)₃Si-O-(CH₃)₂Si-O-Si(CH₃)₃ | (CH₃)₃Si-[O-(CH₃)₂Si]₂-O-Si(CH₃)₃ |
| (CH₃)₃Si-[O-(CH₃)₂Si]₃-O-Si(CH₃)₃ | (CH₃)₃Si-[O-(CH₃)₂Si]₄-O-Si(CH₃)₃ |
| (CH₃)₃Si-[O-(CH₃)₂Si]₅-O-Si(CH₃)₃ | (CH₃)₃Si-[O-(CH₃)₂Si]₆-O-Si(CH₃)₃ |
| (CH₃)₃Si-[O-(CH₃)₂Si]₇-O-Si(CH₃)₃ | (CH₃)₃Si-[O-(CH₃)₂Si]₈-O-Si(CH₃)₃ |
| (CH₃)₃Si-[O-(CH₃)₂Si]₉-O-Si(CH₃)₃ | (CH₃)₃Si-[O-(CH₃)₂Si]₁₀-O-Si(CH₃)₃ |
| (CH₃)₃Si-[O-(CH₃)₂Si]₁₁-O-Si(CH₃)₃ | (CH₃)₃Si-[O-(CH₃)₂Si]₁₂-O-Si(CH₃)₃ |
| (CH₃)₃Si-[O-(CH₃)₂Si]₁₃-O-Si(CH₃)₃ | (CH₃)₃Si-[O-(CH₃)₂Si]₁₄-O-Si(CH₃)₃ |
| (CH₃)₃Si-[O-(CH₃)₂Si]₁₅-O-Si(CH₃)₃ | (CH₃)₃Si-[O-(CH₃)₂Si]₁₆-O-Si(CH₃)₃ |
| (CH₃)₃Si-[O-(CH₃)₂Si]₁₇-O-Si(CH₃)₃ | (CH₃)₃Si-[O-(CH₃)₂Si]₁₈-O-Si(CH₃)₃ |
| (CH₃)₃Si-[O-(CH₃)₂Si]₁₉-O-Si(CH₃)₃ | (CH₃)₃Si-[O-(CH₃)₂Si]₂₀-O-Si(CH₃)₃ |

eingesetzt, wobei (CH₃)₃Si-O-Si(CH₃)₃ und/oder (CH₃)₃Si-O-(CH₃)₂Si-O-Si(CH₃)₃ und/oder (CH₃)₃Si-[O-(CH₃)₂Si]₂-O-Si(CH₃)₃ besonders bevorzugt sind. (CH₃)₃Si-O-(CH₃)₂Si-O-Si(CH₃)₃ ist ein ganz besonders bevorzugtes Öl.

Selbstverständlich können auch Mischungen der o.g. Silikone in den bevorzugten Mitteln enthalten sein.

Besonders bevorzugte erfindungsgemäß einsetzbare Silikone der Formel (Si-1) weisen bei 20°C Viskositäten von 0,2 bis 2 mm²s⁻¹ auf, wobei besagte Silikone mit Viskositäten von 0,5 bis 1 mm²s⁻¹ besonders bevorzugt sind. Die Viskositäten werden bei 20°C mittels Rotationsviskosimetrie bestimmt.
In dem Mittel sind die besagten Öle der Komponente (ii), insbesondere die bevorzugt genannten besagten Öle der Komponente (ii), in Bezug auf das Gesamtgewicht des erfindungsgemäßen Mittels in einer Menge von 0,5 bis 20,0 Gew.-%, insbesondere von 1,0 bis 15,0 Gew.-%, enthalten. substituierte Arylgruppe, eine gegebenenfalls substituierte Aryl-(C₁ bis C₄)-alkylgruppe oder eine (C₂ bis C₆)-Hydroxyalkylgruppe bedeutet, und
R² eine lineare oder verzweigte (C₈ bis C₃₀)-Alkylgruppe, eine lineare oder verzweigte (C₈ bis C₃₀)-Alkenylgruppe, eine gegebenenfalls substituierte Arylgruppe, gegebenenfalls substituierte Aryl-(C₁ bis C₄)-alkylgruppe, eine gegebenenfalls substituierte Aryl-(C₂ bis C₄)-Alkenylgruppe oder eine (C₂ bis C₆)-Hydroxyalkylgruppe bedeutet,
und
(ii) mindestens ein Silikonöl.
   (B): Mittel zur kosmetischen Behandlung keratinhaltiger Fasern, insbesondere menschlichem Haar, enthaltend in einem kosmetisch akzeptablen Träger
(i) mindestens einen Ester der Formel (I-a) 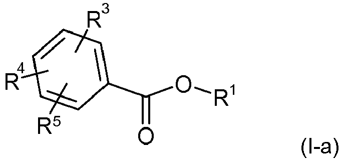 worin
   - R¹: steht für eine lineare oder verzweigte (C₃ bis C₂₄)-Alkylgruppe, eine lineare oder verzweigte (C₆ bis C₂₄)-Alkenylgruppe, eine gegebenenfalls substituierte Arylgruppe, eine gegebenenfalls substituierte Aryl-(C₁ bis C₄)-alkylgruppe oder eine (C₂ bis C₆)-Hydroxyalkylgruppe, und
   - R³, R⁴ und R⁵: stehen unabhängig voneinander für ein Wasserstoffatom, Hydroxy, Amino, (C₁ bis C₄)-alkylamino, Di(C₁ bis C₄)-alkylamino, (C₁ bis C₄)-Alkyl, (C₁ bis C₄)-Alkoxy, (C₁ bis C₄)-Alkoxycarbonyl, oder zwei der Reste bilden gemeinsam mit dem Restmolekül einen fünf- oder sechsgliedrigen Ring
   und
(ii) mindestens ein Silikonöl.
   (C): Mittel zur kosmetischen Behandlung keratinhaltiger Fasern, insbesondere menschlichem Haar, enthaltend in einem kosmetisch akzeptablen Träger
(i) mindestens einen Ester der Formel (I) 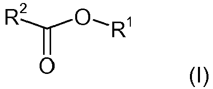 worin
   R¹ eine lineare oder verzweigte (C₂ bis C₃₀)-Alkylgruppe, eine lineare oder verzweigte (C₈ bis C₃₀)-Alkenylgruppe, eine (C₂ bis C₃₀)-Alkylpoly(oxyethylen)gruppe, eine gegebenenfalls substituierte Arylgruppe, eine gegebenenfalls substituierte Aryl-(C₁ bis C₄)-alkylgruppe oder eine (C₂ bis C₆)-Hydroxyalkylgruppe bedeutet, und
   R² eine lineare oder verzweigte (C₈ bis C₃₀)-Alkylgruppe, eine lineare oder verzweigte (C₈ bis C₃₀)-Alkenylgruppe, eine gegebenenfalls substituierte Arylgruppe, gegebenenfalls substituierte Aryl-(C₁ bis C₄)-alkylgruppe, eine gegebenenfalls substituierte Aryl-(C₂ bis C₄)-Alkenylgruppe oder eine (C₂ bis C₆)-Hydroxyalkylgruppe bedeutet,
   und
(ii) mindestens ein Silikonöl der Formel (Si-1)

   (CH₃)₃Si-[O-Si(CH₃)₂]ₓ-O-Si(CH₃)₃ (Si-1),

   enthalten, in der x für eine Zahl von 0 bis 100, vorzugsweise von 0 bis 50, weiter bevorzugt von 0 bis 20 und insbesondere 0 bis 10, steht.
   (D): Mittel zur kosmetischen Behandlung keratinhaltiger Fasern, insbesondere menschlichem Haar, enthaltend in einem kosmetisch akzeptablen Träger
(i) mindestens einen Ester der Formel (I-a) 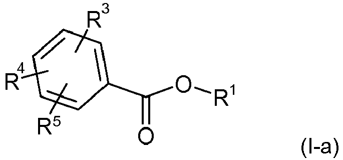 worin
   - R¹: steht für eine lineare oder verzweigte (C₃ bis C₂₄)-Alkylgruppe, eine lineare oder verzweigte (C₆ bis C₂₄)-Alkenylgruppe, eine gegebenenfalls substituierte Arylgruppe, eine gegebenenfalls substituierte Aryl-(C₁ bis C₄)-alkylgruppe oder eine (C₂ bis C₆)-Hydroxyalkylgruppe, und
   - R³, R⁴ und R⁵: stehen unabhängig voneinander für ein Wasserstoffatom, Hydroxy, Amino, (C₁ bis C₄)-alkylamino, Di(C₁ bis C₄)-alkylamino, (C₁ bis C₄)-Alkyl, (C₁ bis C₄)-Alkoxy, (C₁ bis C₄)-Alkoxycarbonyl, oder zwei der Reste bilden gemeinsam mit dem Restmolekül einen fünf- oder sechsgliedrigen Ring
   und
(ii) mindestens ein Silikonöl der Formel (Si-1)

   (CH₃)₃Si-[O-Si(CH₃)₂]ₓ-O-Si(CH₃)₃ (Si-1),

   enthalten, in der x für eine Zahl von 0 bis 100, vorzugsweise von 0 bis 50, weiter bevorzugt von 0 bis 20 und insbesondere 0 bis 10, steht.
   (E): Mittel zur kosmetischen Behandlung keratinhaltiger Fasern, insbesondere menschlichem Haar, enthaltend in einem kosmetisch akzeptablen Träger bezogen auf das Gesamtgewicht des Mittels
(i) 1,0 bis 10,0 Gew.-% mindestens eines Esters der Formel (I) 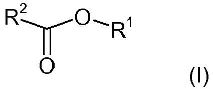 worin
   R¹ eine lineare oder verzweigte (C₂ bis C₃₀)-Alkylgruppe, eine lineare oder verzweigte (C₈ bis C₃₀)-Alkenylgruppe, eine (C₂ bis C₃₀)-Alkylpoly(oxyethylen)gruppe, eine gegebenenfalls substituierte Arylgruppe, eine gegebenenfalls substituierte Aryl-(C₁ bis C₄)-alkylgruppe oder eine (C₂ bis C₆)-Hydroxyalkylgruppe bedeutet, und
   R² eine lineare oder verzweigte (C₈ bis C₃₀)-Alkylgruppe, eine lineare oder verzweigte (C₈ bis C₃₀)-Alkenylgruppe, eine gegebenenfalls substituierte Arylgruppe, gegebenenfalls substituierte Aryl-(C₁ bis C₄)-alkylgruppe, eine gegebenenfalls substituierte Aryl-(C₂ bis C₄)-Alkenylgruppe oder eine (C₂ bis C₆)-Hydroxyalkylgruppe bedeutet,
   und
(ii) 1,0-15,0 Gew.-% mindestens eines Silikonöls.
   (F): Mittel zur kosmetischen Behandlung keratinhaltiger Fasern, insbesondere menschlichem Haar, enthaltend in einem kosmetisch akzeptablen Träger
(i) 1,0 bis 10,0 Gew.-% mindestens eines Esters der Formel (I-a) 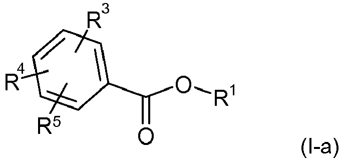 worin
   - R¹: steht für eine lineare oder verzweigte (C₃ bis C₂₄)-Alkylgruppe, eine lineare oder verzweigte (C₆ bis C₂₄)-Alkenylgruppe, eine gegebenenfalls substituierte Arylgruppe, eine gegebenenfalls substituierte Aryl-(C₁ bis C₄)-alkylgruppe oder eine (C₂ bis C₆)-Hydroxyalkylgruppe, und
   - R³, R⁴ und R⁵: stehen unabhängig voneinander für ein Wasserstoffatom, Hydroxy, Amino, (C₁ bis C₄)-alkylamino, Di(C₁ bis C₄)-alkylamino, (C₁ bis C₄)-Alkyl, (C₁ bis C₄)-Alkoxy, (C₁ bis C₄)-Alkoxycarbonyl, oder zwei der Reste bilden gemeinsam mit dem Restmolekül einen fünf- oder sechsgliedrigen Ring
   und
(ii) 1,0-15,0 Gew.-% mindestens eines Silikonöls.
   (G): Mittel zur kosmetischen Behandlung keratinhaltiger Fasern, insbesondere menschlichem Haar, enthaltend in einem kosmetisch akzeptablen Träger
(i) 1,0 bis 10,0 Gew.-% mindestens eines Esters der Formel (I) 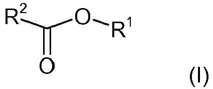 worin
   R¹ eine lineare oder verzweigte (C₂ bis C₃₀)-Alkylgruppe, eine lineare oder verzweigte (C₈ bis C₃₀)-Alkenylgruppe, eine (C₂ bis C₃₀)-Alkylpoly(oxyethylen)gruppe, eine gegebenenfalls substituierte Arylgruppe, eine gegebenenfalls substituierte Aryl-(C₁ bis C₄)-alkylgruppe oder eine (C₂ bis C₆)-Hydroxyalkylgruppe bedeutet, und
   R² eine lineare oder verzweigte (C₈ bis C₃₀)-Alkylgruppe, eine lineare oder verzweigte (C₈ bis C₃₀)-Alkenylgruppe, eine gegebenenfalls substituierte Arylgruppe, gegebenenfalls substituierte Aryl-(C₁ bis C₄)-alkylgruppe, eine gegebenenfalls substituierte Aryl-(C₂ bis C₄)-Alkenylgruppe oder eine (C₂ bis C₆)-Hydroxyalkylgruppe bedeutet,
   und
(ii) 1,0-15,0 Gew.-% mindestens eines Silikonöls der Formel (Si-1)

   (CH₃)₃Si-[O-Si(CH₃)₂]ₓ-O-Si(CH₃)₃ (Si-1),

   enthalten, in der x für eine Zahl von 0 bis 100, vorzugsweise von 0 bis 50, weiter bevorzugt von 0 bis 20 und insbesondere 0 bis 10, steht.
   (H): Mittel zur kosmetischen Behandlung keratinhaltiger Fasern, insbesondere menschlichem Haar, enthaltend in einem kosmetisch akzeptablen Träger
(i) 1,0 bis 10,0 Gew.-% mindestens eines Esters der Formel (I-a) 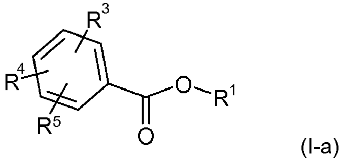 worin
   - R¹: steht für eine lineare oder verzweigte (C₃ bis C₂₄)-Alkylgruppe, eine lineare oder verzweigte (C₆ bis C₂₄)-Alkenylgruppe, eine gegebenenfalls substituierte Arylgruppe, eine gegebenenfalls substituierte Aryl-(C₁ bis C₄)-alkylgruppe oder eine (C₂ bis C₆)-Hydroxyalkylgruppe, und
   - R³, R⁴ und R⁵: stehen unabhängig voneinander für ein Wasserstoffatom, Hydroxy, Amino, (C₁ bis C₄)-alkylamino, Di(C₁ bis C₄)-alkylamino, (C₁ bis C₄)-Alkyl, (C₁ bis C₄)-Alkoxy, (C₁ bis C₄)-Alkoxycarbonyl, oder zwei der Reste bilden gemeinsam mit dem Restmolekül einen fünf- oder sechsgliedrigen Ring
   und
(ii) 1,0-15,0 Gew.-% mindestens eines Silikonöls der Formel (Si-1)

   (CH₃)₃Si-[O-Si(CH₃)₂]ₓ-O-Si(CH₃)₃ (Si-1),

   enthalten, in der x für eine Zahl von 0 bis 100, vorzugsweise von 0 bis 50, weiter bevorzugt von 0 bis 20 und insbesondere 0 bis 10, steht.

Bei den bevorzugten Ausführungsformen, insbesondere den Ausführungsformen (A) bis (H), ist es wiederum bevorzugt, wenn diese als Sprühnebel vorliegen.

Die Mittel enthalten die Inhalts- bzw- Wirkstoffe in einem kosmetisch akzeptablen Träger.

Bevorzugte kosmetisch akzeptable Träger sind alkoholische oder wässrigalkoholische Medien. Letztere umfassen vorzugsweise höchstens 10 Gew.-% Wasser, bezogen auf das gesamte Mittel. Im Rahmen einer bevorzugten Ausführungsform des Mittels enthält das Mittel zusätzlich mindestens einen Alkohol, der 2 bis 6 Kohlenstoffatome und 1 bis 3 Hydroxylgruppen aufweist. Dieser zusätzliche Alkohol wird wiederum bevorzugt ausgewählt aus mindestens einer Verbindung der Gruppe, die gebildet wird aus Ethanol, Isopropanol, Ethylenglykol, 1,2-Propylenglykol, 1,3-Propylenglykol, Glyzerin, n-Butanol, 1,3-Butylenglykol. Ganz besonders bevorzugte Alkohole sind Ethanol, Isopropanol und deren Mischung.

Es ist erfindungsgemäß besonders bevorzugt, wenn der besagte zusätzliche Alkohol mit mindestens 2 bis 6 Kohlenstoffatomen bezogen auf das Gewicht des Mittels in einer Menge von 30,0 bis 70,0 Gew.-%, insbesondere von 40,0 bis 60,0 Gew.-%, enthalten ist.
(K): Mittel zur kosmetischen Behandlung keratinhaltiger Fasern, insbesondere menschlichem Haar, enthaltend bezogen auf das Gesamtgewicht des Mittels
   (i) 1,0 bis 10,0 Gew.-% mindestens eines Esters der Formel (I-a) 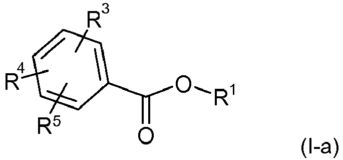 worin
      - R¹: steht für eine lineare oder verzweigte (C₃ bis C₂₄)-Alkylgruppe, eine lineare oder verzweigte (C₆ bis C₂₄)-Alkenylgruppe, eine gegebenenfalls substituierte Arylgruppe, eine gegebenenfalls substituierte Aryl-(C₁ bis C₄)-alkylgruppe oder eine (C₂ bis C₆)-Hydroxyalkylgruppe, und
      - R³, R⁴ und R⁵: stehen unabhängig voneinander für ein Wasserstoffatom, Hydroxy, Amino, (C₁ bis C₄)-alkylamino, Di(C₁ bis C₄)-alkylamino, (C₁ bis C₄)-Alkyl, (C₁ bis C₄)-Alkoxy, (C₁ bis C₄)-Alkoxycarbonyl, oder zwei der Reste bilden gemeinsam mit dem Restmolekül einen fünf- oder sechsgliedrigen Ring
      und
   (ii) 1,0 bis 15,0 Gew.-% mindestens eines Silikonöls, und
   (iii) 30,0 bis 70,0 Gew.-% (insbesondere 40 bis 60 Gew.-%) mindestens eines Alkohols, der 2 bis 6 Kohlenstoffatome und 1 bis 3 Hydroxylgruppen aufweist, insbesondere Ethanol, als Bestandteil des kosmetisch akzeptablen Trägers.
(L): Mittel zur kosmetischen Behandlung keratinhaltiger Fasern, insbesondere menschlichem Haar, enthaltend bezogen auf das Gesamtgewicht des Mittels
   (i) 1,0 bis 10,0 Gew.-% mindestens eines Esters der Formel (I) 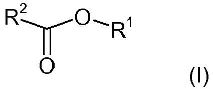 worin
      R¹ eine lineare oder verzweigte (C₂ bis C₃₀)-Alkylgruppe, eine lineare oder verzweigte (C₈ bis C₃₀)-Alkenylgruppe, eine (C₂ bis C₃₀)-Alkylpoly(oxyethylen)gruppe, eine gegebenenfalls substituierte Arylgruppe, eine gegebenenfalls substituierte Aryl-(C₁ bis C₄)-alkylgruppe oder eine (C₂ bis C₆)-Hydroxyalkylgruppe bedeutet, und
      R² eine lineare oder verzweigte (C₈ bis C₃₀)-Alkylgruppe, eine lineare oder verzweigte (C₈ bis C₃₀)-Alkenylgruppe, eine gegebenenfalls substituierte Arylgruppe, gegebenenfalls substituierte Aryl-(C₁ bis C₄)-alkylgruppe, eine gegebenenfalls substituierte Aryl-(C₂ bis C₄)-Alkenylgruppe oder eine (C₂ bis C₆)-Hydroxyalkylgruppe bedeutet,
      und
   (ii) 1,0 bis 15,0 Gew.-% mindestens eines Silikonöls der Formel (Si-1)

      (CH₃)₃Si-[O-Si(CH₃)₂]ₓ-O-Si(CH₃)₃ (Si-1),

      enthalten, in der x für eine Zahl von 0 bis 100, vorzugsweise von 0 bis 50, weiter bevorzugt von 0 bis 20 und insbesondere 0 bis 10, steht
      und
   (iii) 30,0 bis 70,0 Gew.-% (insbesondere 40 bis 60 Gew.-%) mindestens eines Alkohols, der 2 bis 6 Kohlenstoffatome und 1 bis 3 Hydroxylgruppen aufweist, insbesondere Ethanol, als Bestandteil des kosmetisch akzeptablen Trägers.
(M): Mittel zur kosmetischen Behandlung keratinhaltiger Fasern, insbesondere menschlichem Haar, enthaltend bezogen auf das Gesamtgewicht des Mittels
   ( i) 1,0 bis 10,0 Gew.-% mindestens eines Esters der Formel (I-a) 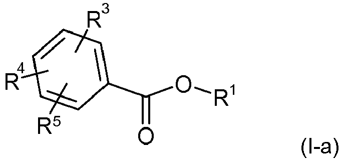 worin
      - R¹: steht für eine lineare oder verzweigte (C₃ bis C₂₄)-Alkylgruppe, eine lineare oder verzweigte (C₆ bis C₂₄)-Alkenylgruppe, eine gegebenenfalls substituierte Arylgruppe, eine gegebenenfalls substituierte Aryl-(C₁ bis C₄)-alkylgruppe oder eine (C₂ bis C₆)-Hydroxyalkylgruppe, und
      - R³, R⁴ und R⁵: stehen unabhängig voneinander für ein Wasserstoffatom, Hydroxy, Amino, (C₁ bis C₄)-alkylamino, Di(C₁ bis C₄)-alkylamino, (C₁ bis C₄)-Alkyl, (C₁ bis C₄)-Alkoxy, (C₁ bis C₄)-Alkoxycarbonyl, oder zwei der Reste bilden gemeinsam mit dem Restmolekül einen fünf- oder sechsgliedrigen Ring
      und
   ( ii) 1,0 bis 15,0 Gew.-% mindestens eines Silikonöls der Formel (Si-1)

      (CH₃)₃Si-[O-Si(CH₃)₂]ₓ-O-Si(CH₃)₃ (Si-1),

      enthalten, in der x für eine Zahl von 0 bis 100, vorzugsweise von 0 bis 50, weiter bevorzugt von 0 bis 20 und insbesondere 0 bis 10, steht
      und
   ( iii) 30,0 bis 70,0 Gew.-% (insbesondere 40 bis 60 Gew.-%) mindestens eines Alkohols, der 2 bis 6 Kohlenstoffatome und 1 bis 3 Hydroxylgruppen aufweist, insbesondere Ethanol, als Bestandteil des kosmetisch akzeptablen Trägers.

Bei den bevorzugten Ausführungsformen (J) bis (M), ist es wiederum bevorzugt, wenn diese als Sprühnebel, insbesondere als mit Hilfe eines Treibmittels erzeugte Sprühnebel, vorliegen.

Als zusätzliche Co-Solventien können organische Lösungsmittel oder ein Gemisch aus Lösungsmitteln mit einem Siedepunkt unter 400°C in einer Menge von 0,1 bis 15 Gewichtsprozent, bevorzugt von 1 bis 10 Gewichtsprozent bezogen auf das gesamte Mittel enthalten sein. Besonders geeignet als zusätzliche Co-Solventien sind unverzweigte oder verzweigte Kohlenwasserstoffe wie Pentan, Hexan, Isopentan und cyclische Kohlenwasserstoffe wie Cyclopentan und Cyclohexan.

Die Mittel können optional zusätzlich mindestens ein festigendes Polymer enthalten. Die optional zugesetzten festigenden Polymere sind bevorzugterweise anionisch, amphoter, zwitterionisch oder nichtionisch, besonders bevorzugt anionisch. Die festigenden Polymere sind üblicherweise in einer Menge von 0,1 Gew.-% bis 20,0 Gew.-%, besonders bevorzugt von 0,2 Gew.-% bis 10,0 Gew.-%, jeweils bezogen auf das Gewicht des Mittels, enthalten.

Unter Polymeren werden erfindungsgemäß Verbindungen verstanden, die von den Verbindungen der Komponenten (i) und (ii) der Mittel verschieden sind und die ein Molekulargewicht von wenigstens 10000 g/mol besitzen. Die Polymere sind aus einer Vielzahl von Molekülen aufgebaut, in denen eine Art oder mehrere Arten von Atomen oder Atomgruppierungen (sogenannte konstitutive Einheiten, Grundbausteine oder Wiederholungseinheiten) wiederholt aneinander gereiht sind. Die Polymere werden durch Polyreaktion erhalten, wobei letztere künstlich (d.h. synthetisch) oder natürlich erfolgen kann.

Unter filmbildenden Polymeren sind solche Polymere zu verstehen, welche beim Trocknen einen kontinuierlichen Film auf der Haut, dem Haar oder den Nägeln hinterlassen. Derartige Filmbildner können in den unterschiedlichsten kosmetischen Produkten wie beispielsweise Gesichtsmasken, Make-up, Haarfestigern, Haarsprays, Haargelen, Haarwachsen, Haarkuren, Shampoos oder Nagellacken verwendet werden. Bevorzugt sind insbesondere solche Polymere, die eine ausreichende Löslichkeit in Wasser oder Wasser/Alkohol-Gemischen besitzen, um in dem erfindungsgemäßen Mittel in vollständig gelöster Form vorzuliegen. Die filmbildenden Polymere können synthetischen oder natürlichen Ursprungs sein. Unter filmbildenden Polymeren werden weiterhin erfindungsgemäß solche Polymere verstanden, die bei Anwendung in 0,01 bis 20 Gew.-%-iger wässriger, alkoholischer oder wässrigalkoholischer Lösung in der Lage sind, auf dem Haar einen transparenten Polymerfilm abzuscheiden.

Festigende Polymere tragen zum Halt und/oder zum Aufbau des Haarvolumens und der Haarfülle der Gesamtfrisur bei. Diese Polymere sind gleichzeitig auch filmbildende Polymere und daher generell typische Substanzen für formgebende Haarbehandlungsmittel wie Haarfestiger, Haarschäume, Haarwachse, Haarsprays. Die Filmbildung kann dabei durchaus punktuell sein und nur einige Fasern miteinander verbinden.

Als eine Testmethode für die festigende Wirkung eines Polymers wird häufig der so genannte curlretention - Test angewendet.

Anionische Polymere sind Polymere, die mindestens eine anionische Gruppe tragen (bevorzugt *-COO⁻ und/oder *-SO₃⁻) und keine kationische Gruppe (z.B. *-N+Me₃ oder *-N+H₃) tragen.

Die anionischen festigenden Polymere sind in dem erfindungsgemäßen Mittel bevorzugt in einer Menge von 0,1 Gew.-% bis 20 Gew.-%, besonders bevorzugt von 0,5 Gew.-% bis 15 Gew.-%, ganz besonders bevorzugt von 1,0 Gew.-% bis 10,0 Gew.-%, am bevorzugtesten von 2,0 Gew.-% bis 6,0 Gew.-%, jeweils bezogen auf das Gewicht der erfindungsgemäßen Zusammensetzung, enthalten.

Erfindungsgemäß bevorzugt verwendbare anionische Polymere haben ein Molekulargewicht von 10000 bis 250000 g/mol, insbesondere von 20000 bis 200000 g/mol.

Es ist erfindungsgemäß bevorzugt, wenn das anionische festigende Polymere mindestens eine Struktureinheit der Formel (S1) enthält, die ausgewählt wird aus mindestens einer Struktureinheit der Formeln (S1-1) und/oder (S1-2) 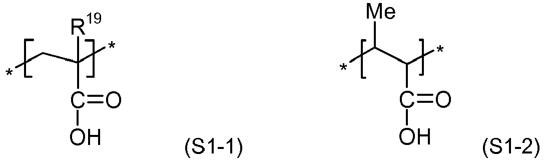 worin R¹⁹ für ein Wasserstoffatom oder eine Methylgruppe steht
und zusätzlich mindestens eine Struktureinheit der Formel (S2) enthält, die ausgewählt wird aus mindestens einer Struktureinheit der Formeln (S2-1) bis (S2-8) 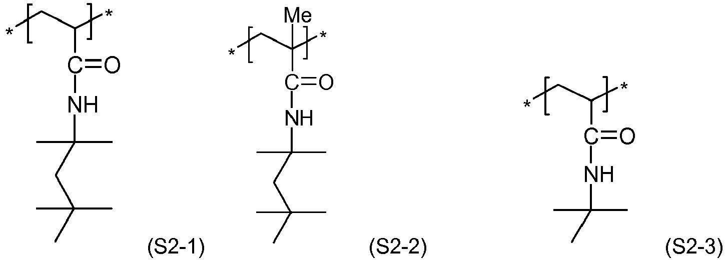 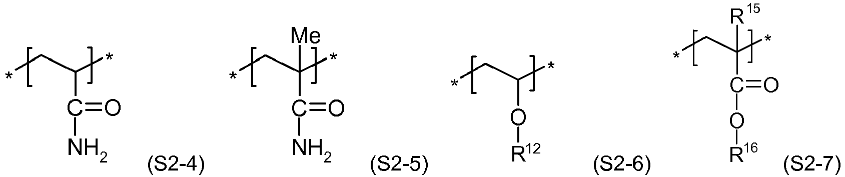 worin
R¹² für eine (C₂ bis C₁₂)-Acylgruppe (insbesondere für Acetyl oder Neodecanoyl), R¹⁵ für ein Wasserstoffatom oder eine Methylgruppe und R¹⁶ für eine (C₁ bis C₄)-Alkylgruppe oder eine (C₂ bis C₄)-Hydroxyalkylgruppe (insbesondere 2-Hydroxyethyl) steht.

Gemäß obiger Formeln und allen folgenden Formeln steht eine chemische Bindung, die mit dem Symbol * gekennzeichnet ist, für eine freie Valenz des entsprechenden Strukturfragments.

Im Rahmen einer Ausführungsform gelten solche Mittel als erfindungsgemäß verwendbar, die als anionisches festigendes Polymer mindestens ein Polymer enthalten, das mindestens eine Struktureinheit der Formel (S1-2) und mindestens eine Struktureinheit der Formel (S2-6) enthält 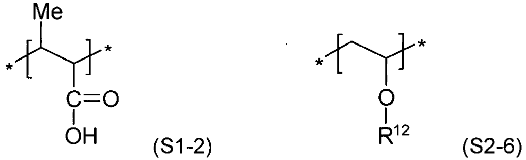 worin R¹² für eine (C₂ bis C₁₂)-Acylgruppe (insbesondere für Acetyl und/oder Neodecanoyl) steht. Besonders bevorzugte Polymere dieser Art werden ausgewählt aus mindestens einem Polymer der Gruppe, die gebildet wird aus
- Copolymeren aus Vinylacetat und Crotonsäure.
- Copolymeren aus Vinylpropionat und Crotonsäure,
- Copolymeren aus Vinylneodecanoat, Vinylacetat und Crotonsäure.

Solche Copolymere werden beispielsweise von der Firma Clariant unter dem Handelsnamen Aristoflex A 60 (INCI-Bezeichnung: VA/Crotonates Copolymer) in einem Isopropanol-WasserGemisch (60 Gew.-% Aktivsubstanz), von der Firma BASF unter dem Handelsnamen Luviset CA 66 (Vinylacetat/Crotonsäure-Copolymer 90:10, INCl-Bezeichnung VA/Crotonates Copolymer) bereitgestellt, von der Firma National Starch unter dem Handelsnamen Resyn 28-2942 bzw. Resyn 28-2930 (INCI-Bezeichnung: VA/Crotonates/Vinyl Neodecanoate Copolymer) bereitgestellt.

Im Rahmen einer bevorzugten Ausführungsform gelten solche Zusammensetzungen als bevorzugt geeignet, die als anionisches festigendes Polymer mindestens ein Polymer enthalten, das mindestens eine Struktureinheit der Formel (S1-1) und mindestes eine Struktureinheit der Formel (S2-7) umfasst 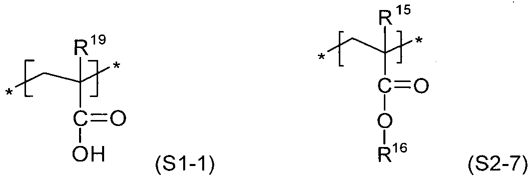 worin
R¹⁵ und R¹⁹ stehen unabhängig voneinander für ein Wasserstoffatom oder eine Methylgruppe R¹⁶ steht für eine (C₁ bis C₄)-Alkylgruppe (insbesondere für eine Methylgruppe oder eine Ethylgruppe) oder eine (C₂ bis C₄)-Hydroxyalkylgruppe (insbesondere für 2-Hydroxyethyl).

Diese Polymere bilden eine Formfixierung, die besonders kompatibel mit der erfindungsgemäßen Kombination der Inhaltsstoffe (i) und (ii) des Mittels ist. Eine Frisur hält ihre Form aufs Beste und weist einen natürlichen Glanz auf.

Ferner ist es wiederum ganz besonders bevorzugt, wenn das anionische festigende Polymer neben den obigen Struktureinheiten der Formeln (S1-1) und (S2-7) zusätzlich mindestens eine Struktureinheit der Formel (S2-3) enthält. 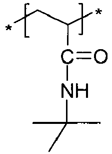 (S2-3). Dabei ist es wiederum bevorzugt, wenn R¹⁹ gemäß Formel (S1-1) für eine Methylgruppe steht. Geeignet erwiesen sich Polymere dieser Art werden ausgewählt aus mindestens einem Polymer der Gruppe, die gebildet wird aus Copolymeren aus Acrylsäure und Ethylacrylat und N-tert.-Butylacrylamid. Solche Copolymere werden beispielsweise von der Firma BASF unter dem Handelsnamen Ultrahold® Strong (INCI-Bezeichnung: Acrylates/t-Butylacrylamide Copolymer, weißes, schüttfähiges Granulat) oder Ultrahold® 8 (INCI-Bezeichnung: Acrylates/t-Butylacrylamide Copolymer, weißes, schüttfähiges Granulat) bereitgestellt.

Ein Mittel einer besonders bevorzugten Ausführungsform enthält als anionisches festigendes Polymer mindestens ein Polymer, das mindestens eine Struktureinheit der Formel (S1-1) und mindestes eine Struktureinheit der Formel (S2-7a) und mindestens eine Struktureinheit der Formel (S2-7b) enthält 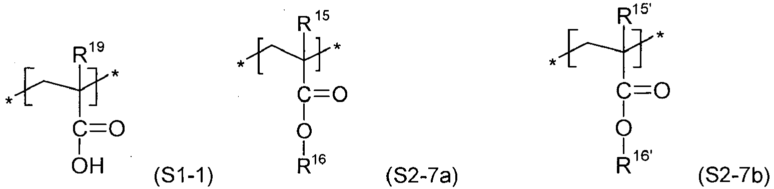 worin
R¹⁵, R^{15'} und R¹⁹ stehen unabhängig voneinander für ein Wasserstoffatom oder eine Methylgruppe, R¹⁶ steht für eine (C₁ bis C₄)-Alkylgruppe (insbesondere für eine Methylgruppe und/oder eine Ethylgruppe und/oder eine Butylgruppe),
R^{16'} steht für eine (C₂ bis C₄)-Hydroxyalkylgruppe (insbesondere für 2-Hydroxyethyl).

Es ist erfindungsgemäß bevorzugt, wenn in Formel (S1-1) R¹⁹ für eine Methylgruppe steht.

Es ist erfindungsgemäß bevorzugt, wenn in Formel (S2-7b) R¹⁵' für eine Methylgruppe und R¹⁶' für eine 2-Hydroxyethylgruppe steht.

Copolymere, die durch die Copolymerisation von Butylacrylat, Methylmethacrylat, Ethylacrylat, Methacrylsäure und 2-Hydroxyethylmethacrylat hergestellt werden, sind erfindungsgemäß besonders bevorzugt.

Dabei sind wiederum Polymere mit der INCI-Bezeichnung Acrylates/Hydroxyalkylesters acrylates Copolymer ganz besonders bevorzugt. Ein solches Polymer ist beispielsweise unter der Handelsbezeichnung Acudyne® 1000 von der Firme Dow erhältlich.

Unter einem amphoteren Polymer wird erfindungsgemäß ein Polymer verstanden, das in einem protischen Lösemittel bei Standardbedingungen Struktureinheiten mit anionischen Gruppen, welche durch Gegenionen unter Erhaltung der Elektroneutralität kompensiert werden müssen, trägt und zusätzlich Struktureinheiten mit durch Protonierung kationisierbaren Gruppen aufweist, jedoch frei von permanent kationisierten Gruppen ist. Unter anionische Gruppen fallen Carboxyl- und Sulfonsäuregruppen. Unter permanent kationisierten Stickstoffatomen sind solche Stickstoffatome zu verstehen, die eine positive Ladung tragen und dadurch eine quartäre Ammoniumverbindung bilden. Per Definition gehören auch N-Oxid-haltige Polymere zu den amphoteren Polymeren.

Es ist erfindungsgemäß geeignet, wenn das zusätzliche amphotere, festigende Polymer mindestens eine Struktureinheit der Formel (S1) enthält, die ausgewählt wird aus mindestens einer Struktureinheit der Formeln (S1-1) bis (S1-3) 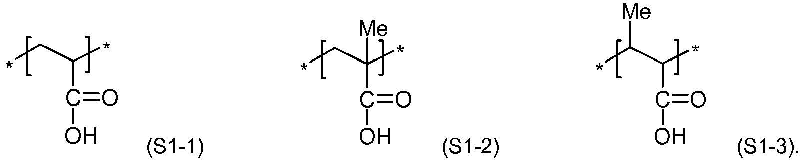 und neben mindestens einer Struktureinheit der Formeln (S1-1) bis (S1-3) zusätzlich mindestens eine Struktureinheit der Formel (S2) enthält, die ausgewählt wird aus mindestens einer Struktureinheit der Formel (S2-9) bis (S2-15) 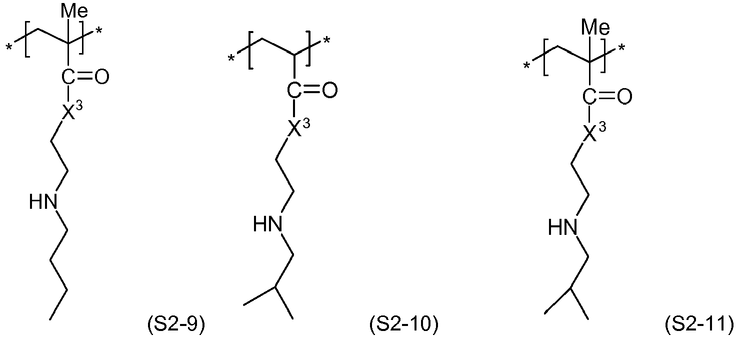 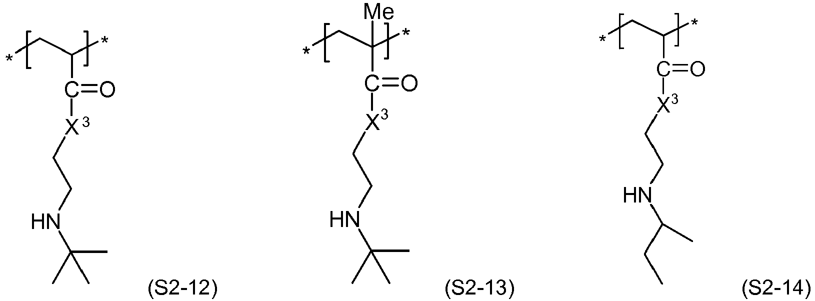 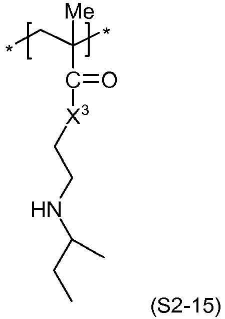 worin
X³ steht für ein Sauerstoffatom oder eine Gruppe NH.

Es ist wiederum erfindungsgemäß geeignet, wenn das amphotere, festigende Polymere neben mindestens einer Struktureinheit der Formeln (S1-1) bis (S1-3) und mindestens einer Struktureinheit der Formeln (S2-9) bis (S2-15) zusätzlich mindestens eine Struktureinheit der Formeln (S2-1) bis (S2-8) umfasst 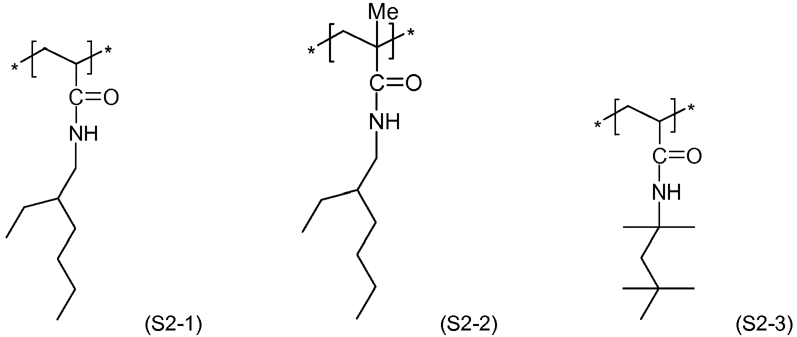 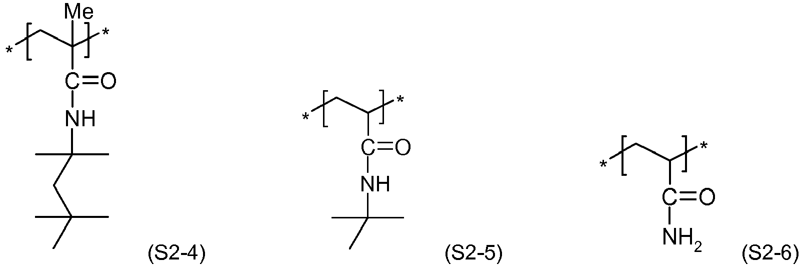 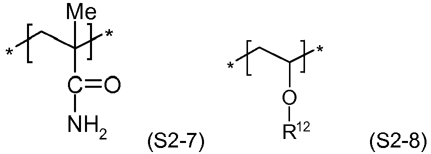 worin
R¹² für eine (C₂ bis C₁₂)-Acylgruppe (insbesondere für Acetyl oder Neodecanoyl) steht.

Bevorzugt eignet sich ein amphoteres, festigendes Polymer, welches mindestens eine Struktureinheit der Formel (S1-1), mindestens eine Struktureinheit der Formel (S2-3) und mindestens eine Struktureinheit der Formel (S2-16) (insbesondere ausgewählt aus der Gruppe, die gebildet wird aus obigen Formeln (S2-5) bis (S2-12) mit der Maßgabe, dass X³ für ein Sauerstoffatom steht), 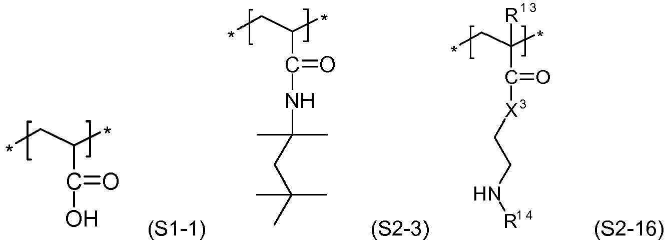
worin X³ steht für ein Sauerstoffatom oder eine Gruppe NH,
R¹³ steht für ein Wasserstoffatom oder eine Methylgruppe und
R¹⁴ steht für eine Alkylgruppe mit 4 Kohlenstoffatomen (insbesondere n-Butyl, sec-Butyl, iso-Butyl oder tert-Butyl).

Dabei ist es wiederum besonders geeignet, wenn das zusätzliche amphotere, festigende Polymere neben den obigen Struktureinheiten der Formeln (S1-1), (S2-3) und (S2-16) zusätzlich mindestens eine Struktureinheit der Formel (S3) enthält 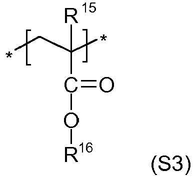 worin
R¹⁵ steht für ein Wasserstoffatom oder eine Methylgruppe
R¹⁶ steht für eine (C₁ bis C₄)-Alkylgruppe (insbesondere eine Methylgruppe oder eine Ethylgruppe).

Zusätzliche amphotere Polymere dieser Art werden ausgewählt aus der Gruppe, die gebildet wird aus Copolymeren aus Acrylsäure, (C₁ bis C₄)-Alkylacrylat, N-(C₄-Alkyl)aminoethylmethacrylat und N-(C₈-Alkyl)acrylamid.

Ein Beispiel eines im Rahmen dieser Ausführungsform verwendbaren amphoteren, festigenden Polymers ist das unter dem Handelsnamen Amphomer® von der Firma National Starch erhältliche Polymer mit der INCI-Bezeichnung Octylacrylamide/Acrylates/Butylaminoethylmethacrylate Copolymer.

Ein weiteres zusätzliches amphoteres festigendes Polymer umfasst
- mindestens ein Monomer A1 ausgewählt aus Acrylsäure, Methacrylsäure, Acrylsäurealkylestern und Methacrylsäurealkylestern, und
- mindestens ein amphoteres Monomer A2 der Formel A2 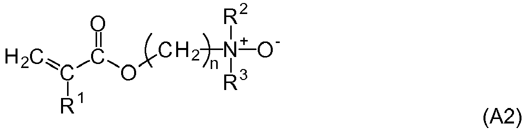 wobei
   - R¹ für H oder CH₃,
   - R² und R³ jeweils unabhängig voneinander für gegebenenfalls verzweigtes C₁₋₁₀-Alkyl und
   - n für eine ganze Zahl von 1 bis 20 steht.

Unter zusätzlichen amphoteren festigenden Polymeren, die aus den genannten Monomeren gebildet sind, werden im Sinne der vorliegenden Ausführungsform der Erfindung nur solche Copolymere verstanden, die neben Polymereinheiten, die aus dem Einbau der genannten Monomere A1 und A2 in das Copolymer resultieren, maximal 5 Gew.-%, vorzugsweise maximal 1 Gew.-%, Polymereinheiten enthalten, die auf den Einbau anderer Monomere zurückgehen. Vorzugsweise sind die Copolymere A ausschließlich aus Polymereinheiten aufgebaut, die aus dem Einbau der genannten Monomere A1 und A2 in das Copolymer resultieren.

Geeignete Monomere A1 sind Acrylsäure, Methacrylsäure, Acrylsäure-C₁₋₂₀-alkylester und Methacrylsäure-C₁₋₂₀-alkylester. Besonders geeignet ist Monomer A1 ausgewählt aus Acrylsäure, Methacrylsäure, Acrylsäuremethylester, Methacrylsäuremethylester, Acrylsäureethylester, Methacrylsäureethylester, Acrylsäurepropylester, Methacrylsäurepropylester, Acrylsäureisopropylester, Methacrylsäureisopropylester, Acrylsäurelaurylester, Methacrylsäurelaurylester, Acrylsäurecetylester, Methacrylsäurecetylester, Acrylsäurestearylester und Methacrylsäurestearylester, ganz besonders bevorzugt aus Acrylsäure, Methacrylsäure, Acrylsäuremethylester, Methacrylsäuremethylester, Acrylsäureethylester, Methacrylsäureethylester, Acrylsäurelaurylester, Methacrylsäurelaurylester, Acrylsäurestearylester und Methacrylsäurestearylester.

Geeignete Monomere A2 sind (Meth)acryloylalkylaminoxide der Formel A2, wobei R² und R³ jeweils unabhängig voneinander für Methyl, Ethyl, Propyl, Isopropyl, Butyl, Isobutyl oder tert.-Butyl, besonders bevorzugt für Methyl stehen. Geeignete Monomere A2 sind weiterhin ausgewählt aus mindestens einem Monomer aus der Gruppe, die gebildet wird aus (Meth)acryloylalkylaminoxiden der Formel A2, wobei n für eine ganze Zahl von 1 bis 5, vorzugsweise für eine ganze Zahl von 1 bis 3 und besonders bevorzugt für 2 steht.

Bevorzugt sind auch Monomere A2 ausgewählt aus mindestens einem Monomer aus der Gruppe, die gebildet wird aus (Meth)acryloylalkylaminoxiden der Formel A2, wobei R¹ für CH₃ steht. Besonders geeignet sind die Monomere A2 ausgewählt aus mindestens einem Monomer aus der Gruppe, die gebildet wird aus (Meth)acryloylalkylaminoxiden der Formel A2, wobei R² und R³ unabhängig voneinander für Methyl, Ethyl, Propyl, Isopropyl, Butyl, Isobutyl oder tert.-Butyl, besonders bevorzugt für Methyl, n jeweils für eine ganze Zahl von 1 bis 5, vorzugsweise für eine ganze Zahl von 1 bis 3 und besonders bevorzugt für 2, und R¹ jeweils für CH₃ steht.
Ganz besonders geeignet ist Monomer A2 ausgewählt aus mindestens einem Monomer aus der Gruppe, die gebildet wird aus (Meth)acryloylalkylaminoxiden der Formel A2, wobei R¹, R² und R³ für CH₃ und n für 2 stehen.

In einer besonders geeigneten Ausführungsform enthält das Mittel mindestens ein amphoteres festigendes Polymer, das gebildet ist aus
- mindestens zwei Monomeren A1, wobei das erste Monomer ausgewählt ist aus Acrylsäure, Methacrylsäure, Acrylsäuremethylester, Methacrylsäuremethylester, Acrylsäureethylester, Methacrylsäureethylester, Acrylsäurepropylester, Methacrylsäurepropylester, Acrylsäureisopropylester und Methacrylsäureisopropylester, und das zweite Monomer ausgewählt ist aus Acrylsäurestearylester und Methacrylsäurestearylester, und
- als Monomer A2 Methacryloylethylaminoxid, insbesondere Methacryloylethyl-N,N-dimethylaminoxid (in Formel (A2): R¹ = CH₃, n = 2, R² und R³ = CH₃).

Auch diese Copolymere sind bekannt und beispielsweise unter der Bezeichnung Diaformer Z-632 von der Firma Clariant erhältlich, wobei der Einsatz von Diaformer Z-632 besonders bevorzugt ist.

In einer geeigneten Ausführungsform enthält das Mittel mindestens ein amphoteres festigendes Polymer, das gebildet ist aus
- mindestens drei Monomeren A1, wobei das erste Monomer ausgewählt ist aus Acrylsäure, Methacrylsäure, Acrylsäuremethylester, Methacrylsäuremethylester, Acrylsäureethylester, Methacrylsäureethylester, Acrylsäurepropylester, Methacrylsäurepropylester, Acrylsäureisopropylester und Methacrylsäureisopropylester, das zweite Monomer ausgewählt ist aus Acrylsäurelaurylester und Methacrylsäurelaurylester, und das dritte Monomer ausgewählt ist aus Acrylsäurestearylester und Methacrylsäurestearylester, und
- als Monomer A2 Methacryloylethylaminoxid, insbesondere Methacryloylethyl-N,N-dimethylaminoxid (in Formel (A2): R¹ = CH₃, n = 2, R² und R³ = CH₃).

Entsprechende Copolymere sind ebenfalls bekannt und beispielsweise unter den Bezeichnungen Diaformer Z-611, Diaformer Z-612, Diaformer Z-613, Diaformer Z-631, Diaformer Z-633, Diaformer Z-651, Diaformer Z-711N, Diaformer Z-712N und Diaformer Z-731 N von der Firma Clariant erhältlich, wobei der Einsatz von Diaformer Z-712N und Diaformer Z-651 bevorzugt ist.

Das Mittel enthält im Rahmen einer weiteren bevorzugten Ausführungsform zusätzlich zum anionischen festigenden Polymer mindestens ein nichtionisches, festigendes Polymer.

Unter einem nichtionischen Polymer wird erfindungsgemäß ein Polymer verstanden, das in einem protischen Lösemittel bei Standardbedingungen keine Struktureinheiten mit permanent kationischen Gruppen oder anionischen Gruppen trägt, welche jeweils durch Gegenionen unter Erhaltung der Elektroneutralität kompensiert werden müssen. Unter kationische Gruppen fallen beispielsweise quaternisierte Ammoniumgruppen jedoch keine protonierten Amine. Unter anionische Gruppen fallen beispielsweise Carboxyl- und Sulfonsäuregruppen.

Die zusätzlichen nichtionischen festigenden Polymere werden günstigenfalls ausgewählt aus mindestens einem Polymer der Gruppe, die gebildet wird, aus
- Homopolymeren und nichtionischen Copolymeren des N-Vinylpyrrolidons,
- nichtionischen Copolymeren des Isobutens,
- nichtionischen Copolymeren des Maleinsäureanhydrids.

Mittel, die als zusätzliches nichtionisches festigendes Polymer mindestens ein Polymer ausgewählt aus der Gruppe, die gebildet wird aus
- Polyvinylpyrrolidon,
- Copolymeren aus N-Vinylpyrrolidon und Vinylestern von Carbonsäuren mit 2 bis 18 Kohlenstoffatomen, insbesondere aus N-Vinylpyrrolidon und Vinylacetat,
- Copolymere aus N-Vinylpyrrolidon und N-Vinylimidazol und Methacrylamid,
- Copolymere aus N-Vinylpyrrolidon und N-Vinylimidazol und Acrylamid,
- Copolymere aus N-Vinylpyrrolidon mit N,N-Di(C₁ bis C₄)-Alkylamino-(C₂ bis C₄)-alkylacrylamid,
oder Gemische aus diesen Polymeren enthalten, sind erfindungsgemäß besonders bevorzugt.

Mittel, die als zusätzliches nichtionisches festigendes Polymer mindestens ein Polymer ausgewählt aus der Gruppe, die gebildet wird aus
- Polyvinylpyrrolidon,
- Copolymeren aus N-Vinylpyrrolidon und Vinylestern von Carbonsäuren mit 2 bis 18 Kohlenstoffatomen, insbesondere aus N-Vinylpyrrolidon und Vinylacetat,
oder Gemische aus diesen Polymeren enthalten, sind erfindungsgemäß ganz besonders bevorzugt.

Geeignete Polyvinylpyrrolidone sind beispielsweise Handelsprodukte wie Luviskol® K 90 oder Luviskol® K 85 der Firma BASF SE.

Kommen Copolymere aus N-Vinylpyrrolidon und Vinylacetat zum Einsatz, ist es wiederum geeignet, wenn das Molverhältnis der aus dem Monomer N-Vinylpyrrolidon enthaltenen Struktureinheiten zu den aus dem Monomer Vinylacetat enthaltenen Struktureinheiten des Polymers im Bereich von 20 zu 80 bis 80 zu 20, insbesondere von 30 zu 70 bis 60 zu 40, liegt.

Geeignete Copolymerisate aus Vinylpyrrolidon und Vinylacetat sind beispielsweise unter dem Warenzeichen Luviskol® VA 37, Luviskol® VA 55, Luviskol® VA 64 und Luviskol® VA 73 von der Firme BASF SE erhältlich.

Weiterhin eignen sich erfindungsgemäß solche Mittel, die mindestens ein nichtionisches festigendes Polymer enthalten, umfassend mindestens eine Struktureinheit der Formel (M-I) und mindestens eine Struktureinheit der Formel (M-IV) 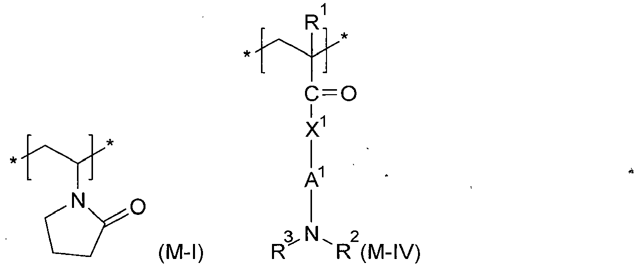 worin
R¹ für ein Wasserstoffatom oder eine Methylgruppe steht,
X¹ für ein Sauerstoffatom oder eine Gruppe NH steht,
A¹ für eine Gruppe Ethan-1,2-diyl, Propan-1,3-diyl oder Butan-1,4-diyl steht
R² und R³ stehen unabhängig voneinander für eine (C₁ bis C₄)-Alkylgruppe.

Dabei ist es insbesondere günstig, wenn das obige nichtionische festigende Polymer ausgewählt wird aus mindestens einem Polymer, welches für Formel (M-IV) mindestens eines oder mehrere der folgenden Merkmale erfüllt:
- R¹ bedeutet eine Methylgruppe,
- X¹ steht für eine Gruppe NH,
- A¹ steht für Ethan-1,2-diyl oder Propan-1,3-diyl,
- R² und R³, stehen unabhängig voneinander für Methyl oder Ethyl, (besonders bevorzugt für Methyl).

Dabei gilt wiederum die Gruppe der Polymere
- Vinylcaprolactam/Vinylpyrrolidon/Dimethylaminoethylmethacrylat-Copolymer (beispielsweise INCI-Bezeichnung: Vinyl Caprolactam/PVP/Di-methylaminoethyl Methacrylate Copolymer unter dem Handelsnamen Gaffix® VC 713 (ISP)),
- Vinylpyrrolidon/Vinylcaprolactam/Dimethylaminopropylmethacrylamid-Copolymer (beispielsweise INCI-Bezeichnung: VP/Vinyl Caprolactam/DMAPA Acrylates Copolymer unter dem Handelsnamen Aquaflex® SF-40 (ISP)),
- Vinylcaprolactam/Vinylpyrrolidon/Dimethylaminoethylmethacrylat-Copolymer (beispielsweise als.35-39% Festkörper in Ethanol in form des Handelsprodukts Advantage LC E mit der INCI-Bezeichnung: Vinyl Caprolactam/VP/Dimethylaminoethyl Methacrylate Copolymer, Alcohol, Lauryl Pyrrolidone (ISP)),
- Vinylpyrrolidon/Dimethylaminopropylmethacrylamid-Copolymer (beispielsweise INCI-Bezeichnung: VP/DMAPA Acrylates Copolymer unter dem Handelsnamen Styleze CC-10 (10 Gew.-% Aktivsubstanz) (ISP)),
als Liste zur Auswahl mindestens eines oder mehrerer zusätzlichen nichtionischer festigender Polymere daraus.

Die Mittel können weiterhin die Hilfs- und Zusatzstoffe enthalten.

Als Pflegestoff kann das Mittel beispielsweise zusätzlich mindestens ein Proteinhydrolysat und/oder eines seiner Derivate enthalten. Proteinhydrolysate pflanzlichen Ursprungs, z. B. Soja-, Mandel-, Erbsen-, Kartoffel- und Weizenproteinhydrolysate, sind beispielsweise unter den Warenzeichen Gluadin® (Cognis), DiaMin® (Diamalt), Lexein® (Inolex), Hydrosoy® (Croda), Hydrolupin® (Croda), Hydrosesame® (Croda), Hydrotritium® (Croda) und Crotein® (Croda) erhältlich.

Die Proteinhydrolysate können in den Mitteln beispielsweise in Konzentrationen von 0,01 Gew.-% bis zu 20 Gew.-%, vorzugsweise von 0,05 Gew.-% bis zu 15 Gew.-% und ganz besonders bevorzugt in Mengen von 0,05 Gew.-% bis zu 5 Gew.-%, jeweils bezogen auf die gesamte Anwendungszubereitung enthalten sein.

Weiterhin kann das Mittel zusätzlich mindestens ein Vitamin, ein Provitamin, eine Vitaminvorstufe und/oder eines derer Derivate enthalten. Dabei sind erfindungsgemäß solche Vitamine, Provitamine und Vitaminvorstufen bevorzugt, die üblicherweise den Gruppen A, B, C, E, F und H zugeordnet werden. Panthenol, Pantolacton, Pyridoxin und seine Derivate sowie Nicotinsäureamid und Biotin sind besonders bevorzugt. In einer bevorzugten Ausführungsform enthalten die Mittel Panthenol, vorzugsweise in einer Menge von 0,1 bis 5 Gew.-%, jeweils bezogen auf das gesamte Mittel.

Weiterhin können die Mittel zusätzlich mindestens einen Pflanzenextrakt enthalten. Erfindungsgemäß sind vor allem die Extrakte aus Grünem Tee, Eichenrinde, Brennnessel, Hamamelis, Hopfen, Henna, Kamille, Klettenwurzel, Schachtelhalm, Weißdorn, Lindenblüten, Mandel, Aloe Vera, Fichtennadel, Rosskastanie, Sandelholz, Wacholder, Kokosnuss, Mango, Aprikose, Limone, Weizen, Kiwi, Melone, Orange, Grapefruit, Salbei, Rosmarin, Birke, Malve, Wiesenschaumkraut, Quendel, Schafgarbe, Thymian, Melisse, Hauhechel, Huflattich, Eibisch, Meristem, Ginseng und Ingwerwurzel bevorzugt.

Weitere Wirk-, Hilfs- und Zusatzstoffe, sind beispielsweise
- Strukturanten wie Maleinsäure und Milchsäure,
- Parfümöle, Dimethylisosorbid und Cyclodextrine,
- Entschäumer wie Silikone,
- Farbstoffe zum Anfärben des Mittels,
- Substanzen zur Einstellung des pH-Wertes, wie beispielsweise übliche Säuren, insbesondere Genusssäuren, und Basen,
- Konsistenzgeber wie Zuckerester, Polyolester oder Polyolalkylether,
- Komplexbildner wie EDTA, NTA, β-Alanindiessigsäure und Phosphonsäuren,
- Quell- und Penetrationsstoffe wie Propylenglykolmonoethylether, Carbonate, Hydrogencarbonate, Guanidine, Harnstoffe sowie primäre, sekundäre und tertiäre Phosphate,
- Konservierungsmittel,
- Antioxidantien.

Bezüglich weiterer fakultativer Komponenten sowie der eingesetzten Mengen dieser Komponenten wird ausdrücklich auf die dem Fachmann bekannten einschlägigen Handbücher verwiesen.

Die Formulierung der Mittel kann in allen für üblichen Formen erfolgen, beispielsweise in Form von Lösungen, die als Lotion oder Pump- oder Aerosolspray auf das Haar aufgebracht werden können, oder anderen Zubereitungen, die für die Anwendung auf dem Haar geeignet sind.

Vorzugsweise werden die Mittel als Pumpspray oder insbesondere als Aerosolspray ausgestaltet.

Bei der Ausgestaltung als Pumpspray liegen die Mittel in einem Nichtaerosolbehälter mit Versprühvorrichtung vor. Für die Ausgestaltung als Aerosolspray umfassen die erfindungsgemäßen Mittel zusätzlich mindestens ein Treibmittel und werden in einem Druckgasbehälter ("Aerosolbehälter") mit Versprüheinrichtung konfektioniert.

Die Druckgasbehälter, mit deren Hilfe ein Produkt durch den inneren Gasdruck des Behälters über ein Ventil verteilt wird, bezeichnet man definitionsgemäß als "Aerosolbehälter". Als "Nichtaerosolbehälter" wird im Umkehrschluß zur Aerosoldefinition ein Behältnis unter Normaldruck definiert, mit dessen Hilfe ein Produkt mittels mechanischer Einwirkung durch ein Pumpsystem verteilt wird.

Insbesondere bevorzugt liegen die Mittel als Sprühnebel vor. Zu diesem Zweck ist es wiederum bevorzugt die Mittel als Aerosolspray auszugestalten. Bevorzugte Mittel enthalten daher besonders bevorzugt zusätzlich mindestens ein Treibmittel.
Mittel, die in Form eines Aerosolprodukts vorliegen, lassen sich in üblicher Art und Weise herstellen. In der Regel werden alle Bestandteile des Mittels mit Ausnahme des Treibmittels in einen geeigneten druckfesten Behälter eingefüllt. Dieser wird daraufhin mit einem Ventil verschlossen. Über herkömmliche Techniken wird schließlich die gewünschte Menge Treibmittel eingefüllt.

In der Ausführungsform als Aerosolspray sind erfindungsgemäß geeignete Treibmittel beispielsweise ausgewählt aus N₂O, Dimethylether, CO₂, Luft, Alkanen mit 3 bis 5 Kohlenstoffatomen, wie Propan, n-Butan, iso-Butan, n-Pentan und iso-Pentan, und deren Mischungen. Bevorzugt sind Dimethylether, Propan, n-Butan, iso-Butan und Mischungen daraus. Gemäß einer bevorzugten Ausführungsform werden die genannten Alkane, Mischungen der genannten Alkane oder Mischungen der genannten Alkane mit Dimethylether als einziges Treib mittel eingesetzt. Die Erfindung umfasst aber ausdrücklich auch die Mitverwendung von Treibmitteln vom Typ der Fluorchlorkohlenwasserstoffe, insbesondere aber der Fluorkohlenwasserstoffe.

Das Treibmittel ist in den Mitteln der Ausführungsform als Aerosolspray bevorzugt in einer Menge von 30 bis 60 Gew.-%, insbesondere von 35 bis 55 Gew.-% - bezogen auf das Gewicht des gesamten Mittels - enthalten.

Ganz besonders bevorzugt werden Mischungen von Propan und Butan als alleiniges Treibmittel verwendet im Gewichtsverhältnis Propan zu Butan von 70 zu 30 bis 10 zu 90. Diese Mischungen werden wiederum bevorzugt in den erfindungsgemäßen Mitteln in einer Menge von 30 bis 55 Gew.-% - bezogen auf das Gewicht des gesamten Mittels - eingesetzt. Unter Butan wird erfindungsgemäß n-Butan, iso-Butan und Gemische aus n-Butan und iso-Butan verstanden.

Über das Mengenverhältnis von Treibmittel zu den übrigen Bestandteilen der Zubereitungen lassen sich bei gegebener Sprühvorrichtung die Größen der Aerosoltröpfchen und die jeweilige Größenverteilung einstellen.

Die Sprührate der erfindungsgemäßen Sprays beträgt bevorzugt 5,0 bis 15,0 g/10s, besonders bevorzugt 6,0 bis 10,0 g/10 s.

Die Mittel und Produkte, die diese Mittel enthalten, insbesondere Aerosolhaarsprays, zeichnen sich insbesondere dadurch aus, dass sie behandeltem Haar einen sehr natürlichen Glanz verleihen. Ferner ist es bevorzugt, wenn nach der Applikation des Mittels das Mittel in den keratinhaltigen Fasern verbleibt, d.h. nicht wieder ausgespült wird.

Ferner sind solche Verfahren bevorzugt, bei denen eine Anwendung eines Trockenshampoos, enthaltend mindestens ein Sorbens (insbesondere Stärke oder deren Derivate (wie bevorzugt Reisstärke und/oder Stärke Ocetenyl Succinat) und/oder Silika) und/oder eines kosmetischen Mittels, enthaltend mindestens ein fixierend wirkendes Polymer, der Applikation des kosmetischen Mittels unmittelbar vorausgegangen ist.

### Beispiele

Die folgenden Mengenangaben verstehen sich - soweit nichts anderes vermerkt ist - als Gewichtsprozent.

Folgende Rezepturen wurden durch Vermischen der angegebenen Rohstoffe mit Ausnahme des Treibmittels bereitgestellt und in eine Aerosolsprühdose abgefüllt. Die Aerosoldosen wurden mit dem Ventil verschlossen und abschließend das entsprechende Treibmittel durch das Ventil zugefügt:

| Rohstoffe | A | B | C |
|---|---|---|---|
| Dimethicone | - | - | 2,50 |
| Octamethyltrisiloxan | 5,00 | 10,00 | 2,50 |
| Benzoesäure C₁₂-C₁₅-Alkylester | 2,50 | 5,00 | 5,00 |
| Parfum | 0,10 | 0,10 | 0,10 |
| Treibmittel (Mischung aus 15% Propan, 85% Butan) | 50,00 | 50,00 | 50,00 |
| Ethanol, vergällt | ad 100 | ad 100 | ad 100 |

Es wurde die Wirkung der erfindungsgemäßen Mittel A, B und C auf unvorbehandelter Haarsträhne und auf mit einem Trockenshampoo vorbehandelter Haarsträhne getestet. Nach Sprühapplikation auf das unvorbehandelte Haar bewirkten die erfindungsgemäßen Mittel einen natürlichen Glanz. Das Haar war locker, unbeschwert und ließ sich leicht kämmen. Gleiches wurde beobachtet, wenn das Haar mit folgendem Aerosol-Trockenshampoo durch aufsprühen, 10 Minuten einwirken und auskämmen vorbehandelt wurde:

| Rohstoff | Gew.-% |
|---|---|
| Reisstärke | 5,00 |
| Ethanol | 10,00 |
| Parfum | 0,10 |
| Butan | ad 100 |

## Patentansprüche

1. Verwendung eines Mittels, enthaltend bezogen auf das Gesamtgewicht des Mittels
(i) 1,0 bis 10,0 Gew.-% mindestens eines Esters Benzoesäure mit einem C12 bis C15-Alkanol,
und
(ii) 1,0 bis 15,0 Gew.-% mindestens eines Silikonöls der Formel (Si-1) (CH₃)₃Si-[O-Si(CH₃)₂]ₓ-O-Si(CH₃)₃ (Si-1), enthalten, in der x für eine Zahl von 0 bis 20 steht und
(iii) 30,0 bis 70,0 Gew.-% Ethanol, als Bestandteil des kosmetisch akzeptablen Trägers.
zur Erzeugung von Glanz auf menschlichen Haaren.

2. Aerosolspray, enthaltend ein in einem Aerosolbehälter mit Versprühvorrichtung konfektioniertes kosmetisches Mittel des Anspruchs 1, wobei das kosmetische Mittel zusätzlich mindestens ein Treibmittel umfasst.

## Claims

1. The use of an agent containing, based on the total weight of the agent,
(i) 1.0 to 10.0 wt.% of at least one ester of benzoic acid having a C12 to C15 alkanol, and
(ii) 1.0 to 15.0 wt.% of at least one silicone oil of formula (Si-1) (CH₃)₃Si-[O-Si(CH₃)₂]ₓ-O-Si(CH₃)₃ (Si-1), in which x is a number from 0 to 20, and
(iii) 30.0 to 70.0 wt.% ethanol, as a component of the cosmetically acceptable carrier, for producing shine on human hair.

2. An aerosol spray containing a cosmetic agent of claim 1 that is packaged in an aerosol container having a spray device, wherein the cosmetic agent additionally comprises at least one propellant.

## Revendications

1. Utilisation d'un agent contenant, par rapport au poids total de l'agent,
(i) 1,0 à 10,0 % en poids d'au moins un ester de l'acide benzoïque avec un alcanol en C₁₂ à C₁₅,
et
(ii) 1,0 à 15,0 % en poids d'au moins une huile de silicone de formule (Si-1) (CH₃)₃Si-[O-Si(CH₃)₂]ₓ-O-Si(CH₃)₃ (Si-1), dans laquelle x représente un nombre de 0 à 20
et
(iii) 30,0 à 70,0 % en poids d'éthanol, comme ingrédient du support cosmétiquement acceptable,
pour conférer un éclat aux cheveux.

2. Spray aérosol, contenant un agent cosmétique selon la revendication 1 conditionné dans une bombe aérosol dotée d'un dispositif de pulvérisation, l'agent cosmétique comprenant en plus au moins un agent propulseur.
